# EUROPEAN PATENT APPLICATION

(11) **EP 2 468 283 A1**
(43) Date of publication of application: **27.06.2012**
(21) Application number: 10015591.0
(22) Date of filing: 14.12.2010
(51) Int. Cl.: A61K 31/7004, A61K 33/00

(54) **Composition for treating or preventing cell degeneration**

(71) Applicant: Garuti, Eliseo, 33312 Villaviciosa Asturias (ES)
(72) Inventor: Garuti, Eliseo, 33312 Villaviciosa Asturias (ES)
(74) Representative: Barberi, Vittorio

(57) **Abstract**

The invention relates to a composition for the treatment and the prevention of cell degeneration characterized in that it is constituted by a compound of Potassium Bicarbonate and D-Ribose.

## Description

The present invention relates to a drug usable for the prevention and the reduction of tissue degeneration; in particular, the drug is particularly useful in cases of degenerative diseases caused by particular conditions of stress, such as, for example. the exposure to ionizing radiations (gamma rays and X-rays), exposure to UV. or cellular damage caused by toxic molecules, etc...

It is known that radiations in general, as well as other factors, produce damage at the cellular level by way of chemical oxidations of lipids and proteins, resulting in oxidative stress. The same angiogenesis, triggering off a hyperoxygenation of the new cytoplasmatic capillary vessels is itself a source of oxidizing stress. The general effects thus produced in the organism are mainly the following: a weakening of the immune system; a stepped-up physiological organic deterioration; the production of degenerative forms.

A particularly significant example of radiations are those relating to the radiotherapy. And it is well known that radiotherapy is designed to stop tumor growth by maximizing the ratio of dose to the tumor and normal tissues. Ionizing radiations on normal cells can induce apoptosis or damages which may cause the progression of cancer. It is therefore important and essential to prevent and reduce the effects of exposure to healthy tissue during radiation therapy.

Among the effects of exposure to radiation or, in general, to oxidative stress, are considered the bio-physical and chemical changes that can result in damage to cell membranes and to the DNA. In fact, ] In fact, in the presence of oxidizing stress, the NA+ - K+ pump does not perform its task correctly and there occurs an imbalance of the four fundamental cations which are needed to maintain cellular functioning, namely sodium, potassium, calcium and magnesium. Especially in the presence of degenerative diseases and in particular neoplastic forms, Sodium NA + (a prevalent cation in extracellular fluids) tends to substitute Potassium K+ inside the cell, with a resulting serious imbalance and alteration of the NA+ - K+ pump.

Aim of the present invention is to eliminate the above mentioned drawbacks providing with a drug having the characteristics of claim 1. Other characteristics are object of the dependent claims.

Among the advantages of the present invention there is that the drug is a very powerful anti-oxidizing that reduces the effect of free radicals; that it strengthens the activity of the immune system maintaining or revitalizing the concentration of intracellular Potassium to the required levels; that it ties together the functional characteristics of its components giving rise to results surprisingly better in respect to the components singularly used; that it is completely atoxic (at prescribed doses): that it can be used over a long period of time; that it have a very simple posology.

Every technician who works in this field will better understand these advantages and features and further advantages and features of the present invention thanks to the enclosed figures relating to preliminary results of the effects of the drug in question on human cells; in the figures:
- Fig. 1 relates to a photo of a control sample;
- Fig 2 relates to a photo of a control sample irradiated with X-ray;
- Fig 3 relates to a photo of a sample treated with the drug of the present invention and irradiated with X-ray;
- Fig 4 relates to a photo of a sample treated with the drug of the present invention and not irradiated.

The following description illustrates the preliminary results of the effects of D-ribose and KHCO₃ in an aqueous solution on HTB125 irradiated with X-ray.

In particular, the study was directed to the effects of D-Ribose and KHCO₃ in an aqueous solution on normal human breast cells HTB125 irradiated with X-ray.

As mentioned earlier, the radiotherapy is aimed at stopping tumor growth, maximizing the ratio of dose to the tumor and normal tissues. The ionizing radiations on normal cells can induce apoptosis or damages that may cause the progression of cancer. It is therefore important and essential to prevent and reduce the effects of exposure to healthy tissue during radiation therapy.

### Materials and Methods.

HTB125 cells of mammary gland not tumoral were purchased from American Type Culture Collection (ATCC) - Manassas. VA, USA. The cells were maintained in DMEM (Dulbeccos's Modified Eagle's Medium) purchased from Lonza to which are added 10% fetal calf serum (Lonza), 1% L-glutamine (Sigma Aldrich), 1% Penicillin-Streptomycin (Sigma Aldrich) and 30 µg/m) of epidermal growth factor (Gibco). The cells were incubated at 37°C in a humidified atmosphere with 5% CO₂.

The D-ribose was purchased from Sigma-Aldrich and potassium bicarbonate from BDH Prolabo. A stock solution (K:D-Rib) 250mM was prepared by dissolving 0.1mg of D-Ribose and 0.3mg of KHCO₃, the whole being stirred until the CO₂ is completely released into the air.

The cells were maintained changing the culture media every 48 hours.

The cell population was splitted when it reaches 90-100% of confluence. To split the cell cultures that reached confluence, was uses trypsin (Sigma Aldrich). This enzyme acts as a protease capable of separating both the links between cell and substrate as cell-cell.

### Treatment and irradiation of cell cultures.

The day before irradiation, cells are seeded 8000/ml and treated with a solution of 5mM (K: D-Rib). The solution was obtained by diluting the stock 250mM (K: D-Rib) in DMEM. The day after seeding cells were irradiated.

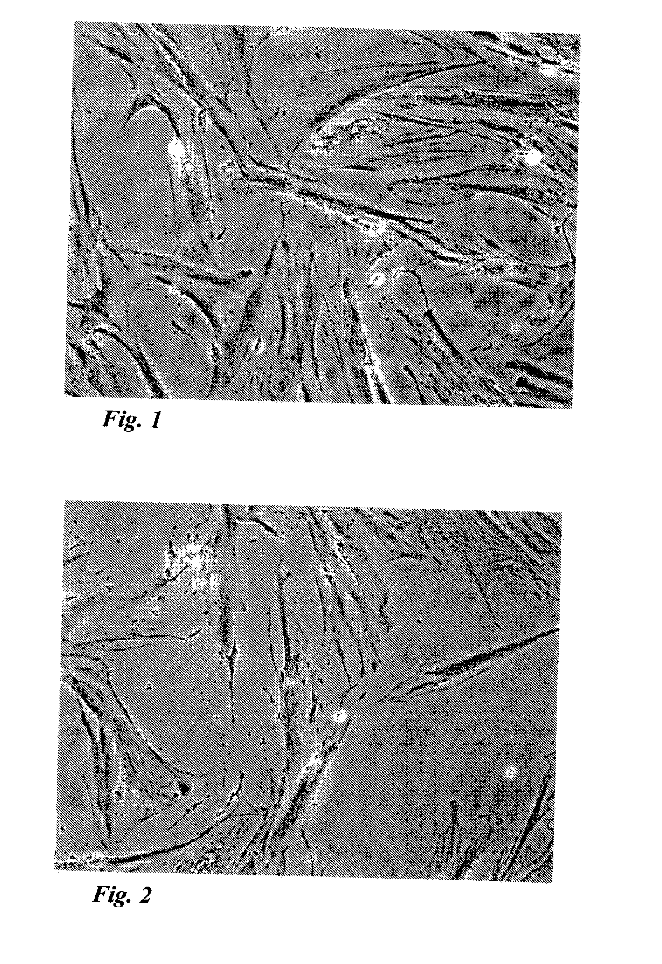

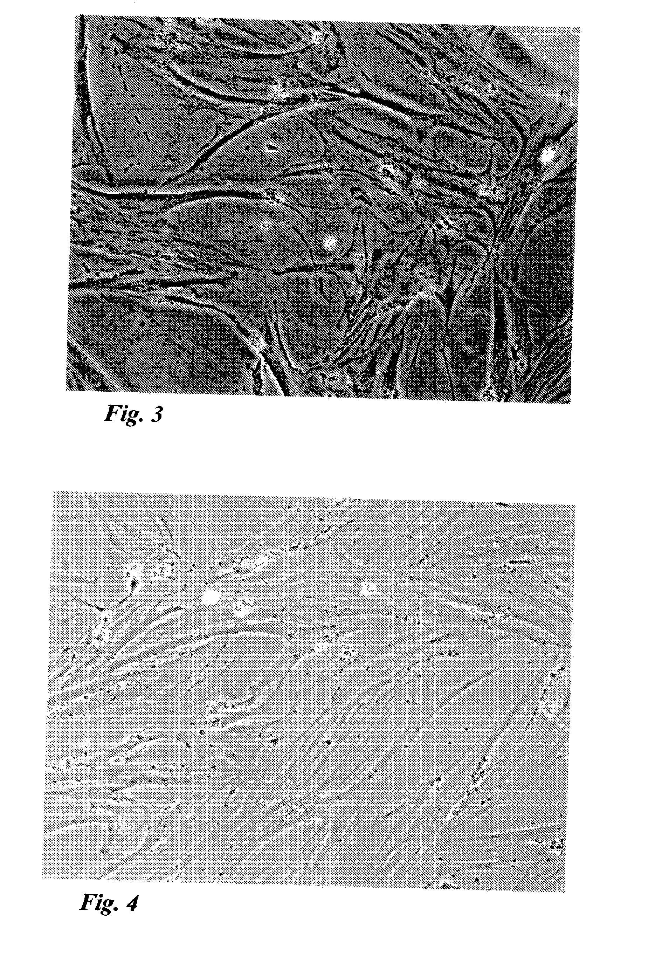

In particular, four samples were prepared: control (C, shown in Fig.1), irradiated control (C_irr, shown in Fig.2), treated non-irradiated (Krib, shown in Fig.4) and treated irradiated (Krib_irr, shown in Fig.3). The irradiation was performed with an X-ray tube (Faxitron cabinet 43855D) by placing the Petri dish on a rotating plate for ensuring uniformity of dose. The X-ray tube was set to 106kV and 3mA. The dose was equivalent to 1.01Gy/min and the radiation occurred for a time of 2 min. The evaluation of the absorbed dose was carried out via the system Victoreen NERO mAx Model 8000. In addition, the dose was measured by simulating the Petri system covering the sensor with the cover of the same Petri dish. The day after irradiation have changed the means of treated and untreated samples. The treatment was carried out until the end of the experiment.

### Results - Discussion.

To study cell growth and the effect of treatment, the cell growth was controlled and in particular the time between irradiation and the first split, i.e. the attainment of confluence.

Samples C, Krib and Krib_irr have reached 90% confluence after 7 days after sowing while the sample C_irr 10 days after sowing. In addition, the use of trypsin was much less effective in the samples C, Krib and Krib_irr, compared to the sample C_irr.

These results show that the samples C. Krib and Krib_irr have the same growth kinetics. The kinetics in Krib_irr treated with 5mM (K: D-Rib) was not altered by the radiation: on the contrary the sample C_irr has reached the confluence with a significant delay of 3 days. Considering the difference in efficacy of trypsin, it can be assumed a protective effect of the solution (K:D-Rib) on not tumoral cells HTB125 treated before irradiation and subsequently the same.

The drug of the invention, which in its preferred form of administration is injectable. consists of a mixture of Potassium Bicarbonate (KHCO₃) and an antioxidant agent that includes D-ribose (C₅H₁₀O₅).

In another embodiment, the compound object of this invention is a salt derived from D-ribose and is obtained by combining Potassium Bicarbonate and D - ribose in pure crystalline form. The compound thus obtained, which is called Potassium Ribosate, is water soluble and can be injected intramuscularly.

There are no known other trials in which it was administered intramuscularly the composition which constitutes the drug of the present invention, nor are known other trials that suggest the use of a drug injection in which the components of Potassium Bicarbonate and D - Ribose provide results so surprising in the prevention and treatment of degenerative diseases.

It is known that D Ribose is a molecule in which the hydroxyl O-H, in the reaction which forms the Potassium Ribosate, is substituted by the O-K group.

Ribose salifies easily with alkaline bicarbonates dissolved in cold water and with warm earthy-alkaline carbonates at a temperature of 45-50°C, and working out of CO₂. By cold evaporation, under high vacuum, crystallized salts (Ribosates) are obtained. Potassium Ribosate is a pure microcrystalline easily water-soluble white salt and somewhat unstable due to its easy oxidizability. It is secured through the salification of D-Ribose in cold water solution with Potassium bicarbonate. Its action does not produce any toxicity (given the stated dosages) and can be used for a long time. Potassium Ribosate can take on two isomeric configurations: the oenolic form and the furanoid form. When in solution it assumes the latter form.

Potassium Ribosate is a very powerful anti-oxidizing, completely atoxyic that can be used over a long period of time (at prescribed doses), acts in a way that reduces the effect of free radicals, strengthening the activity of the immune system and maintaining or revitalizing the concentration of intracellular potassium to the required levels. It is therefore a most valid alimentary integrator and a very powerful cellular anti-oxidizer which ties together the functional characteristics of Ribose and of Potassium, manifesting itself as more active than the two constituents separately taken. The blood haemoglobin contains pyrrolic rings in its molecule; equally pyrrole derivates must be considered many fundamental amino acids. Also of note is the fact that black pigments, skin, hair, moles or birth-marks, etc are closely related to pyrrole black spots, which allows the assumption that said pigments are oxidized compounds and polycondensed compounds having a pyrrolic structure.

Pyrrole, Thiophene and Furan are similar to each other; in the formation of their compounds they follow Angeli's rule of analogies. It is therefore reasonable to assume that during the biological synthesis process of protein derivatives from such compounds there may occur analogous chemical and physiological reactions and that under particular conditions a pyrrolic group may be replaced by a similar thiophenic or furanoid group.

It is important to note that the hydrogen present in the pyrrolic structure can be easily replaced by the potassium cation but not by the sodium potion (the Camician effect), regardless of the physical and chemical affinity between these two.

Both potassium haemoglobinate and potassium proteinate contain pyrrolic structures which can be salified with Potassium Ribosate KHCO₃ and Potassium Ribosate contains in its molecule a furanosic group which by analogy may replace one of the pyrrolic groups of potassium haemoglobinate and proteinate.

This is of the utmost importance because at the beginning of a degenerative process, particularly of neoplastic type, the pyrrolic group are inactive and it is quite likely that the starting point of a degenerative disease is the peptide molecule containing pyrrolic groups at the RNA level. It is assumed that the opening of the pyrrolic molecule (the Camician effect) may give rise to a triggering off of RNA polymerization, this being the beginning of the biological phase of a neoplasia.

Therefore Potassium Ribosate, in reactivating the pyrrolic group, restores the structuring phenomena of the cellular auto-synthesis to the required physiological normality.

Besides, since salification is a reversible process, Potassium Ribosate, carried by haemoglobin and introduced into the cell, re-establishes the equilibrium amongst the intermolecular forces of the peptide groups which are present inside the cell membrane and brings back (or maintains) the required levels of the intracellular potassium concentration.

Finally, the compound Potassium Ribosate protects the cell from the effects of free radicals, precisely by virtue of its anti-oxidizing characteristics.

Potassium Ribosate is especially active against degenerative disease since, as explained earlier, when confronted by a degenerative process, it corrects the process of cellular metabolism by inhibiting its progress and giving it back the equilibrium of its functions with satisfactory results.

It emerges from stoichiometric calculations that the proportion between Potassium Bicarbonate and Ribose must be of a 2 - 1 relation, but it is possible a 3-1 relation. Regarding the storage and the dosage, the compound of Potassium Ribosate must be protected from humidity and the sun's rays and dosed in bags, vials, phials or other suitable separated containers for preparing an extemporary solution of very pure D Ribose and Bicarbonate of Potassium according to the following preferable proportions. The proportions provide to use a bag or a phial of Potassium bicarbonate containing two doses of same and a bag, a vial or a phial of D-Ribose containing a dose of same. It is possible to use three doses of Potassium Bicarbonate and a dose of D-Ribose.

In practice, the details may still vary in an equivalent amount, percentage, type of components used, without going beyond the scope of the idea of the solution adopted and therefore remaining within the limits of the protection afforded by this patent.

## Claims

1. Composition for the treatment and the prevention of cell degeneration **characterized in that** it is constituted by a compound of Potassium Bicarbonate and D-Ribose.

2. Composition according to claim 1, **characterized in that** it comprises two parts of Potassium Bicarbonate and a part of D-Ribose.

3. Composition according to claim 1, **characterized in that** it comprises three parts of Potassium Bicarbonate and a part of D-Ribose.

4. Composition according to claim 1, **characterized in that** it comprises four parts of Potassium Bicarbonate and a part of D-Ribose.

5. Composition according to one of the preceding claims, **characterized in that** it is used to form an intramuscularly injectable drug.

6. Composition according to one of the preceding claims, **characterized in that** the components of said compound are in single doses for each component, associable at the time of use, in order to obtain an extemporaneous solution of a drug.
